# EUROPEAN PATENT SPECIFICATION

(11) **EP 2 818 195 B1**
(45) Date of publication and mention of the grant of the patent: **22.05.2019**
(21) Application number: 13173924.5
(22) Date of filing: 27.06.2013
(51) Int. Cl.: A61M 16/06, A61M 16/08

(54) **PEDIATRIC RESPIRATORY MASK WITH BALL HEAD CONNECTOR**
PÄDIATRISCHE ATEMMASKE MIT KUGELKOPFVERBINDER
MASQUE RESPIRATOIRE PÉDIATRIQUE AVEC RACCORD À TÊTE SPHÉRIQUE

(43) Date of publication of application: 31.12.2014
(73) Proprietor: Air Liquide Medical Systems, 92160 Antony (FR)
(72) Inventor: Alberici, Luca, 25128 BRESCIA (IT); Masserdotti, Fulvio, 25075 BRESCIA (IT); Sandoni, Giuseppe, 25124 BRESCIA (IT)
(74) Representative: Pittis, Olivier

(56) References cited:
- EP-A1- 2 428 244
- EP-A1- 2 510 823
- WO-A1-01/32250
- US-A- 3 824 999
- US-A1- 2006 201 514
- US-A1- 2007 163 600
- US-A1- 2007 272 169
- US-A1- 2008 210 241
- US-A1- 2010 229 866
- US-A1- 2011 232 649
- US-A1- 2012 067 344
- US-A1- 2012 285 452

## Description

The invention concerns a pediatric respiratory mask, in particular a pediatric nasal mask, for use in the treatment of respiratory conditions or diseases affecting infants, children or the like, which comprises a rotating connector with a ball-head for connecting the gas feeding line to the mask body.

Nasal masks are commonly used for delivering non-invasive positive pressure ventilation (NPPV) or for nasal continuous positive airway pressure (N-CPAP) therapy in disordered breathing conditions, such as obstructive sleep apnea (OSA), chronic obstructive pulmonary disease (COPD)...

Nasal masks deliver a flow of breathable gas for, or to assist in, patient respiration. Such a mask assembly typically comprises a rigid or semi-rigid hollow shell, usually made of polymer, defining a breathing chamber that receives at least a part of the patient's nose and further comprising a soft face-contacting cushion that comes into contact with the patient's face and conforms to the various facial contours of the patient face, which cushion is usually made of soft, resilient elastomeric material, such as soft silicone or a similar material, and a forehead support and a headgear for correctly positioning, maintaining and/or securing the mask on the head of a patient.

The forehead support is usually arranged on an expansion part of the mask body forming a holding arm that projects upwardly from the mask body and in the direction of the forehead of the user when the mask is positioned on the user's face This holding arm is also usually made of a a rigid or semi-rigid polymer material.

The mask assembly is secured to the wearer's head by straps or similar devices thereby forming a headgear that can be adjusted to pull the mask against the face with sufficient force to achieve a gas tight seal between the mask and the wearer's face as taught by documents EP-A-462701, EP-A-874667, EP-A-1972357 or WO-A-00/57942.

The hollow shell or mask body typically receives the gas from a gas feeding system comprising a gas supply line which conveys a respiratory gas, such as air under pressure, and a connector element for fluidly connecting the gas supply line to the mask body or shell, so as to deliver the respiratory gas into the breathing chamber of the shell. An example of a nasal mask of this kind is given by EP-A-462701.

The gas feeding line can lead to gas leaks due to its weight and position, which makes the mask less stable and decreases the tightness between the cushion and the face of the patient. This problem occurs particularly on pediatric mask because children move a lot when they sleep.

Attempts have been made to overcome these problems.

Thus document EP-A-2114500 proposes a nasal mask useable with infants and children, which includes a gas feeding system comprising a flexible cushion with a tube connection portion at one or both sides adjacent the patient's nares thereby allows the patient to sleep on their face more comfortably.

EP-A-2428244 teaches a respiratory mask for the mechanical ventilation of premature babies and infants. This respiratory mask has: a nose receiving space to receive at least partially the nose of the patient, defined by a wall made of soft and deformable material, wherein an edge of the wall is integrally formed with the wall and serves to seal the mask against the patient's face; and an antechamber, integrally formed with the nose receiving space and connected to it via a gas exchange opening, the antechamber comprising a ball-socket joint with an outer joint element formed as a shell and an inner oint element in the form of a ball, received in the outer joint element and pivotably movable with respect to it, the inner joint element having a gas inlet for delivering gas to the patient and a gas outlet for extraction of exhalation gas. The inner and outer joint elements may be made of the same material. They can also be made of different materials, and in particular the inner joint element may be made of hard plastic, and the outer joint element may be of a more flexible material. The mask has three holding arms for attachment of attachment straps, one of the arms extending vertically upwards from the mask, the other two extending laterally in opposite directions along the same axis.

US-A-2010/229866, US-A-2012/067344 and WO-A-01/32250 teach various respiratory masks comprising body, arms and tubings for providing gas. The masks are molded in one-piece or comprise several pieces secured together.

US-A-2011/232649, US-A-3824999 and US-A-2007/272169 disclose various respiratory masks comprising ball-head connectors.

However, the current pediatric masks are not totally satisfactory, especially existing pediatric nasal masks.

Hence, the problem to be solved is to provide a pediatric respiratory mask comprising an improved gas feeding system providing more freedom of motion to the patient and ensuring an improved stability, when the mask is worn by the patient, including overnight, especially when the patient is an infant, a child, a toddler or the like.

The solution of the present invention concerns a pediatric respiratory nasal mask comprising :
- a flexible mask body comprising an internal chamber and an inlet orifice in fluid communication with the internal chamber;
- a rigid tubular connector element comprising a) an internal gas passage traversing said connector element, and b) a rigid ball head, said rigid connector element being connected to the inlet orifice of the mask body so that the internal gas passage of the connector element is in fluid communication with the internal chamber of the mask body;
- a holding arm arranged on the mask body and projecting upwardly from said mask body;
- a cushion having an aperture for receiving at least part of the patient's nose, when the patient wears the mask; and
- two lateral arms arranged on each side of the mask body and projecting laterally from said mask body,
wherein:
- the mask body, the holding arm, the cushion and the two lateral arms are made of one single piece of a resilient soft material, the cushion comprising at least one flexible membrane forming a skirt delimiting the aperture, said membrane being made of said resilient soft material and being molded in one piece with the rest of the cushion,
- the mask body has a generally triangular tridimensional shape, and the mask is dimensioned for fitting to the nasal region of an infant or a child, and
- a rigid annular element comprising a rigid polymer is arranged in the inlet orifice of the mask body, said inlet orifice being arranged at the center of the mask body, and the rigid ball head of the connector element being rotatably fixed to the rigid annular element arranged in the inlet orifice.

The pediatric mask according to the present invention can further comprise one or more of the following additional features:
- the rigid polymer of the annular element comprises plastic such as Nylon™.
- the tubular connector element has an elbow shape.
- the tubular connector element comprises venting holes.
- the tubular connector element comprises a secondary inlet orifice carried by an inlet port and in fluid communication with the internal gas passage traversing said connector element.
- a headgear comprising straps.
- one of the two lateral arms projects from the right side of the mask body, while the other of the two lateral arms projects from the left side of the mask body.
- the resilient soft material forming the single piece is silicone.
- the holding arm projecting upwardly from said mask body comprises at least one slot at its terminal end.
- at least one of the two opposite lateral arms projecting laterally from said mask body comprises a slot.
- it further comprises a headgear and connecting elements for fixing the headgear to the mask body.
- the connecting elements comprise one or several straps, one or several straps of the headgear cooperating with one of the slots arranged at the terminal end of the holding arm and/or in at least one of the two lateral arms.
- the two opposite lateral arms have a generally curved-shape, preferably the two lateral arms are curved toward the rear side of the mask body.
- the cushion comprises an aperture having a triangular form or similar shape.

The invention also concerns an assembly comprising a gas delivery device, such as a medical ventilator, and a respiratory mask according to the present invention, preferably the gas delivery device is connected to the respiratory mask by means of a gas line, such as a flexible hose.

A preferred embodiment of a nasal mask according to the present invention is shown in the enclosed Figures, among which :
- Figure 1 represents a front view of an embodiment of a pediatric nasal mask according to the present invention,
- Figure 2 is a rear view of the mask of Figure 1, and
- Figure 3 is a lateral view of the mask of Figure 1 shows the tubing elements fixed to the mask.

In the medical field, flexible gas hoses or lines are usually attached by straps or similar retention elements to the clothes of children of one or several years and of infants/babies of several days, weeks or months in order to avoid or to minimize the risk that the flexible conduct winds around their necks.

This renders the entire conduct system rather rigid and can be a huge problem with children, infants or the like that receive a gaseous treatment delivered by means of a respiratory mask because, as children move their heads, the mask is often displaced due to the traction exerted by the gas hose/line on the mask, while the child moves, and gas leaks appear, thereby creating a discomfort for the patients and decreasing the efficiency of the gaseous treatment.

In other words, the tubing elements, such as the gas conduct, line or hose feeding the respiratory gas to the mask and the connector linking the gas conduct to the mask body can be a problem as it is not always possible, with the existing masks, to get a suitable orientation of said tubing elements vis-à-vis the mask body, especially when the mask is worn by children or the like, leading to a bad positioning of the mask on the patient's face and subsequent gas leaks.

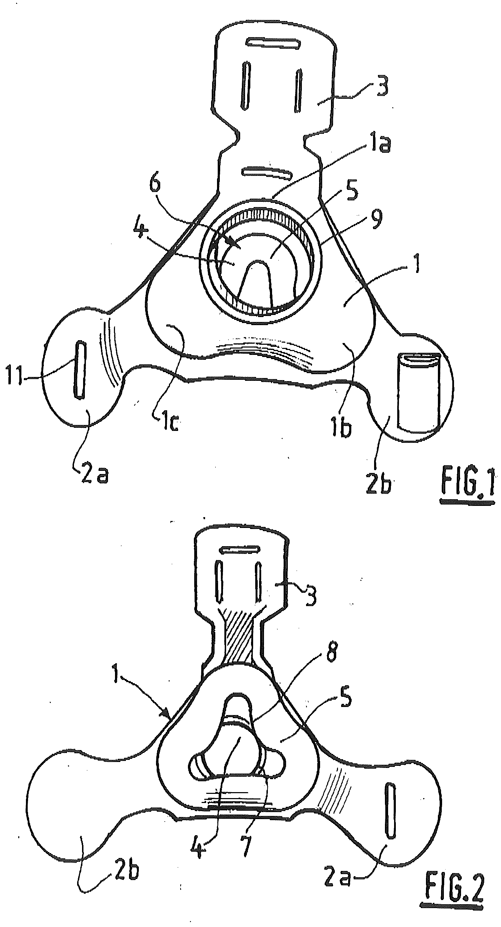

In order to overcome those problems, the present invention proposes a new type of pediatric nasal mask with improved tubing elements, as illustrated in Figures 1 to 3.

The nasal mask of Figures 1 to 3 is especially configured to a pediatric usage.

More precisely, a pediatric respiratory mask according to the present invention comprises a hollow shell or mask body 1 defining an internal breathing chamber 6 or volume, wherein respiratory gas, such as air under pressure, is introduced via an inlet port 4 to which is connected a gas feeding line 13, such as a flexible hose or line, by means of a tubular connector 12. The structure of connector 12 is described below.

The gas inlet orifice 4 is arranged at the center of front side of the mask body 1 and through its wall thereby allowing gas under pressure to be introduced in the breathing chamber 6, said inlet orifice 4 being in fluid communication with the internal chamber 6.

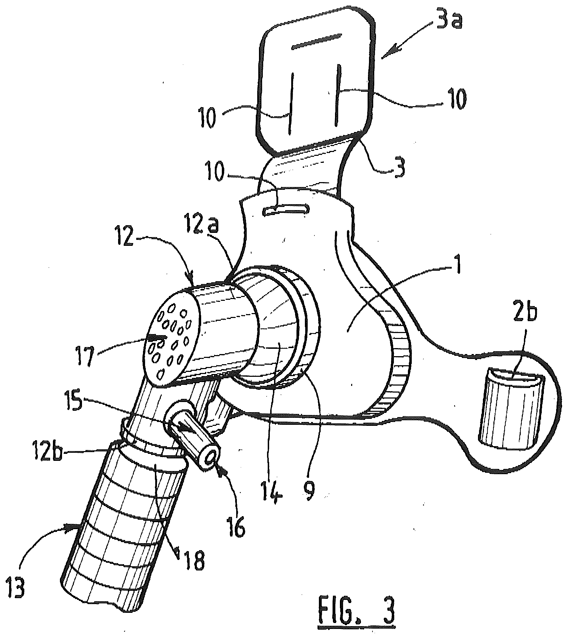

The mask body 1 has a generally triangular tridimensional shape as visible in Figures 1 and 3 so as to better match the nasal regions of the patient.

The mask body 1 receives at least a part of the patient's nose, when said patient is an infant/baby or a child that introduces his/her nose into the internal volume of the breathing chamber 6 of the mask body 1 for breathing, i.e. inhaling, the gas contained therein.

Further, the mask body 1 also comprises, a holding arm 3 forming an upper or frontal support that is integral with the mask body 1 and projecting upwardly from said mask body 1 and, two lateral arms or wings 2a, 2b that are also integrally arranged on the mask body 1 and that project laterally, i.e. on each opposite lateral sides (right and left sides) from said mask body 1 so as to constitute right and left cheek supports, when the mask is worn by a patient.

The holding arm 3 comprises forehead pillows or similar cushioning elements that come in contact with the forehead of the patient, when he/she wears the mask.

Furthermore, a cushion 5 having an aperture 7 for receiving at least part of the patient's nose, when the patient wears the mask, is arranged on the rear side of the mask body 1 as shown in Figure 3.

According to the present invention, in order to provide efficient gas tightness (seal) and/or increased comfort for the patient, when said patient is a infant or child, the mask body 1, the holding arm 3, the cushion 5 and the two lateral arms 2a, 2b are made of a single piece of a resilient soft material, such as silicone. Preferably, they are molded in one piece. Said single piece of resilient soft material being entirely flexible, it can be adapted to a great variety of facial morphologies, especially of various nasal regions of infant or child patients.

The soft resilient cushion 5 that comes into contact with the patient's face, during use of the mask, comprises a central aperture 7 for receiving at least a part of the patient's nose. Said central aperture 7 can have a triangular or similar shape or structure as illustrated in Figure 1-3, so as to match the contours of the nasal region of the patient.

A soft flexible membrane 8 forming a kind of skirt delimits said central aperture 7. Said soft flexible membrane 8 is molded in one piece with the rest of the cushion 5 so as to form part of the single piece of flexible material.

The pediatric mask according to the present invention is a nasal mask. This means that the cushion 5 and the membrane 8 comprise an upper nasal bridge region, a lower region and two lateral regions (i.e. left and right regions) connecting the nasal bridge and lower regions. When the mask is worn by the patient, the upper nasal bridge region is in contact with the nasal bridge of the patient, the lower region is in contact with the area between the nose and the upper lip of the patient and the lateral opposite regions are in contact with the flange regions of the nose of the patient.

Further, the cushion 5 can comprise several membranes 8, such as two superimposed membranes. Using several membranes may improve the gas tightness. Nevertheless, in the present embodiment, a single membrane is provided.

Further, the mask body 1 has a generally triangular tridimensional shape that more or less matches or corresponds to the general structure of a nose. Said generally triangular tridimensional shape or structure comprises three angle areas 1a-1c forming the three corners of the triangular structure of the mask body 1. The holding arm 3 projects upwardly from one 1a of the three angle areas 1a-1c of said mask body 1, whereas the two lateral arms 2a, 2b project laterally from the two other angle areas 1b, 1c of said mask body 1 as illustrated in Figure 1.

Furthermore, the holding arm 3 comprises one or several slots 10 or similar traversing orifices, preferably located at its terminal end 3a, and one (or more) of the two lateral arms 2a, 2b also comprises a slot 11 for receiving the straps of a headgear so that one or several straps of the headgear cooperates with the slots 10, 11 arranged on the holding arm 3 and in at least one of the two lateral arms 2a, 2b in such a way that a headgear comprising straps can be connected to the mask body 1, thereby maintaining the mask in a desired position on the head of the patient during its use and thus obtaining an efficient treatment of respiratory disorders.

The shell or mask body 1 is fluidly linked to a gas supply line 13, such as a flexible hose, by means of the tubular hollow connector 12, which is traversed by an internal gas passage so as to deliver a respiratory gas, such as air under pressure, into the breathing chamber 6 of the mask body 1, said gas flowing through the internal gas passage. The gas supply line 13 is fed with gas under pressure by a respiratory device or ventilator (not shown).

Preferably, the tubular hollow connector 12 has an elbow shape as illustrated in Figure 3.

The tubular hollow connector 12 is fixed to the mask body 1 by means of an annular element 9, such a polymer (plastic) ring or similar, that is arranged in the inlet orifice 4 of the mask body 1 that is located in the front side of the mask body.

The tubular hollow connector 12 comprises, at its proximal end 12a, a ball head 14 and, at its distal end 12b, a connecting part 18 that receives and holds the gas supply line 13, such as a flexible hose made of plastic or similar.

The ball head 14 of the tubular hollow connector 12 is rotatably fixed to the rigid annular element 9 arranged in the inlet orifice 4.

In other words, the ball head 14 cooperates in rotation with the rigid annular element 9 so as to allow a suitable orientation of the hollow connector 12 with respect to the mask body 1, whatever the position of the patient's face.

The annular element 9 comprises or is formed of a rigid polymer, such as plastic, preferably Nylon™.

The tubular connector element 13 has preferably a generally elbow shape as illustrated in Figure 3.

Further, the tubular connector element 12 comprises venting holes 17 for venting to the atmosphere CO₂-enriched gases expired by the patient during expiration phases.

Furthermore, the tubular connector element 12 comprises a secondary inlet orifice 16 carried by an inlet port 15, which is in fluid communication with the internal gas passage traversing said connector element. This secondary inlet orifice 16 can be used as oxygen delivery port or as sampling port.

The pediatric nasal mask of the present invention can be used in a method for treatment of a respiratory disorder or condition affecting an infant and child patient, for example, in non-invasive positive pressure ventilation (NPPV) or in a nasal continuous positive airway pressure (N-CPAP) therapy of sleep disordered breathing (SDB) conditions, such as, for example, obstructive sleep apnea (OSA).

## Claims

1. Pediatric respiratory nasal mask comprising:
- a flexible mask body (1) comprising an internal chamber (6) and an inlet orifice (4) in fluid communication with the internal chamber (6);
- a rigid tubular connector element (12, 12a, 12b) comprising a) an internal gas passage traversing said connector element (12, 12a, 12b), and b) a rigid ball head (14), said rigid connector element (12, 12a, 12b) being connected to the inlet orifice (4) of the mask body (1) so that the internal gas passage of the connector element (12, 12a, 12b) is in fluid communication with the internal chamber (6) of the mask body (1);
- a holding arm (3) arranged on the mask body (1) and projecting upwardly from said mask body (1);
- a cushion (5) having an aperture (7) for receiving at least part of the patient's nose, when the patient wears the mask; and
- two lateral arms (2a, 2b) arranged on each side of the mask body (1) and projecting laterally from said mask body (1),
wherein:
- the mask body (1), the holding arm (3), the cushion (5) and the two lateral arms (2a, 2b) are made of one single piece of a resilient soft material, the cushion (5) comprising at least one flexible membrane (8) forming a skirt delimiting the aperture (7), said membrane (8) being made of said resilient soft material and being molded in one piece with the rest of the cushion (5),
- the mask body (1) has a generally triangular tridimensional shape, and the mask is dimensioned for fitting to the nasal region of an infant or a child, and
- a rigid annular element (9) comprising a rigid polymer is arranged in the inlet orifice (4) of the mask body (1), said inlet orifice (4) being arranged at the center of the mask body (1), and the rigid ball head (14) of the connector element (12, 12a, 12b) being rotatably fixed to the rigid annular element (9) arranged in the inlet orifice (4).

2. Mask according to Claim 1, **characterized in that** the rigid polymer is plastic.

3. Mask according to any one of the preceding Claims, **characterized in that** the tubular connector element (12, 12a, 12b) has an elbow shape and/or comprises venting holes (17).

4. Mask according to any one of the preceding Claims, **characterized in that** the tubular connector element (12, 12a, 12b) comprises a secondary inlet orifice (16) carried by an inlet port (15) and in fluid communication with the internal gas passage traversing said connector element (12, 12a, 12b).

5. Mask according to any one of the preceding Claims, **characterized in that** the generally triangular tridimensional shape of the mask body (1) comprises three angle areas (1a-1c) forming the three corners of the triangular shape of the mask body (1), the holding arm (3) projecting upwardly from one (1a) of said three angle areas (1a-1c) of said mask body (1), and the two lateral arms (2a, 2b) project laterally from the two other angle areas (1b,1c) of said mask body (1).

6. Mask according to any one of the preceding Claims, **characterized in that** the resilient soft material is silicone.

7. Mask according to any one of the preceding Claims, **characterized in that** the aperture (7) of the cushion (5) has a triangular form.

8. Mask according to any one of the preceding Claims, **characterized in that** the membrane (8) and the cushion (5) comprise an upper nasal bridge region, a lower region and two lateral regions connecting the nasal bridge and lower regions so that, when the mask is worn by the patient, the upper nasal bridge region is in contact with the nasal bridge of the patient, the lower region is in contact with the area between the nose and the upper lip of the patient, and the lateral opposite regions are in contact with the flange regions of the nose of the patient.

9. Mask according to any one of the preceding Claims, **characterized in that** it further comprises a headgear comprising straps.

10. Assembly comprising a gas delivery device and a pediatric respiratory mask according to any one of the preceding Claims.

11. Assembly according to Claim 10, **characterized in that** the gas delivery device is connected to the pediatric respiratory mask by means of a gas line (13).

## Patentansprüche

1. Pädiatrische Nasen-Atemmaske, umfassend:
- einen biegsamen Maskenkörper (1), der eine innere Kammer (6) und eine Einlassöffnung (4) in Fluidkommunikation mit der inneren Kammer (6) umfasst;
- ein starres rohrförmiges Verbindungselement (12, 12a, 12b), umfassend a) einen inneren Gasdurchlass, der das Verbindungselement (12, 12a, 12b) durchquert, und b) einen starren Kugelkopf (14), wobei das starre Verbindungselement (12, 12a, 12b) so mit der Einlassöffnung (4) des Maskenkörpers (1) verbunden ist, dass der innere Gasdurchlass des Verbindungselements (12, 12a, 12b) sich in Fluidkommunikation mit der inneren Kammer (6) des Maskenkörpers (1) befindet;
- einen Haltearm (3), der auf dem Maskenkörper (1) angeordnet ist und vom Maskenkörper (1) nach oben vorspringt;
- ein Kissen (5), das eine Öffnung (7) zur Aufnahme mindestens eines Teils der Nase des Patienten aufweist, wenn der Patient die Maske trägt; und
- zwei seitliche Arme (2a, 2b), die auf jeder Seite des Maskenkörpers (1) angeordnet sind und vom Maskenkörper (1) seitlich vorspringen,
wobei:
- der Maskenkörper (1), der Haltearm (3), das Kissen (5) und die beiden seitlichen Arme (2a, 2b) aus einem einzigen Stück aus einem nachgiebigen weichen Material bestehen, wobei das Kissen (5) wenigstens eine biegsame Membran (8) umfasst, die eine die Öffnung (7) abgrenzende Schürze bildet, die Membran (8) aus dem nachgiebigen weichen Material besteht und mit dem Rest des Kissens (5) als ein Teil geformt ist,
- der Maskenkörper (1) eine im Allgemeinen dreieckige dreidimensionale Form aufweist und die Maske zur Anbringung in der Nasenregion eines Säuglings oder eines Kindes bemessen ist, und
- ein starres ringförmiges Element (9), das ein starres Polymer umfasst, in der Einlassöffnung (4) des Maskenkörpers (1) angeordnet ist, wobei die Einlassöffnung (4) in der Mitte des Maskenkörpers (1) angeordnet ist und der starre Kugelkopf (14) des Verbindungselements (12, 12a, 12b) an dem in der Einlassöffnung (4) angeordneten starren ringförmigen Element (9) drehbar befestigt ist.

2. Maske nach Anspruch 1, **dadurch gekennzeichnet, dass** es sich beim starren Polymer um Kunststoff handelt.

3. Maske nach einem der vorstehenden Ansprüche, **dadurch gekennzeichnet, dass** das rohrförmige Verbindungselement (12, 12a, 12b) eine Winkelform aufweist und/oder Entlüftungsbohrungen (17) umfasst.

4. Maske nach einem der vorstehenden Ansprüche, **dadurch gekennzeichnet, dass** das rohrförmige Verbindungselement (12, 12a, 12b) eine sekundäre Einlassöffnung (16) umfasst, die von einem Einlassanschluss (15) getragen wird und sich in Fluidkommunikation mit dem inneren Gasdurchlass befindet, der das Verbindungselement (12, 12a, 12b) durchquert.

5. Maske nach einem der vorstehenden Ansprüche, **dadurch gekennzeichnet, dass** die im Allgemeinen dreieckige dreidimensionale Form des Maskenkörpers (1) drei Winkelbereiche (1a-1c) umfasst, welche die drei Ecken der dreieckigen Form des Maskenkörpers (1) bilden, wobei der Haltearm (3) von einem (1a) der drei Winkelbereiche (1a-1c) des Maskenkörpers (1) nach oben vorspringt und die beiden seitlichen Arme (2a, 2b) von den beiden anderen Winkelbereichen (1b,1c) des Maskenkörpers (1) seitlich vorspringen.

6. Maske nach einem der vorstehenden Ansprüche, **dadurch gekennzeichnet, dass** es sich beim nachgiebigen weichen Material um Silikon handelt.

7. Maske nach einem der vorstehenden Ansprüche, **dadurch gekennzeichnet, dass** die Öffnung (7) des Kissens (5) eine Dreiecksform aufweist.

8. Maske nach einem der vorstehenden Ansprüche, **dadurch gekennzeichnet, dass** die Membran (8) und das Kissen (5) eine obere Nasenrückenregion, eine untere Region und zwei seitliche, den Nasenrücken- und die untere Region verbindende Regionen umfassen, sodass, wenn die Maske vom Patienten getragen wird, die obere Nasenrückenregion sich in Kontakt mit dem Nasenrücken des Patienten befindet, die untere Region sich in Kontakt mit dem Bereich zwischen der Nase und der Oberlippe des Patienten befindet und die seitlichen gegenüberliegenden Regionen sich in Kontakt mit den Flanschregionen der Nase des Patienten befinden.

9. Maske nach einem der vorstehenden Ansprüche, **dadurch gekennzeichnet, dass** sie weiter eine Bänder umfassende Kopfbedeckung umfasst.

10. Anordnung, umfassend eine Gaszuführvorrichtung und eine pädiatrische Atemmaske nach einem der vorstehenden Ansprüche.

11. Anordnung nach Anspruch 10, **dadurch gekennzeichnet, dass** die Gaszuführvorrichtung mittels einer Gasleitung (13) mit der pädiatrischen Atemmaske verbunden ist.

## Revendications

1. Masque respiratoire nasal pédiatrique comprenant :
- un corps de masque souple (1) comprenant une chambre interne (6) et un orifice d'entrée (4) en communication fluidique avec la chambre interne (6) ;
- un élément de raccordement tubulaire rigide (12, 12a, 12b) comprenant a) un passage à gaz interne traversant ledit élément de raccordement (12, 12a, 12b) et b) une tête sphérique rigide (14), ledit élément de raccordement rigide (12, 12a, 12b) étant raccordé à l'orifice d'entrée (4) du corps de masque (1) de sorte que le passage à gaz interne de l'élément de raccordement (12, 12a, 12b) soit en communication fluidique avec la chambre interne (6) du corps de masque (1) ;
- un bras de support (3) agencé sur le corps de masque (1) et faisant saillie vers le haut dudit corps de masque (1) ;
- un coussinet (5) ayant une ouverture (7) pour recevoir au moins une partie du nez du patient lorsque le patient porte le masque ; et
- deux bras latéraux (2a, 2b) agencés sur chaque côté du corps de masque (1) et faisant saillie latéralement dudit corps de masque (1),
dans lequel :
- le corps de masque (1), le bras de support (3), le coussinet (5) et les deux bras latéraux (2a, 2b) sont constitués d'une seule pièce d'un matériau élastique souple, le coussinet (5) comprenant au moins une membrane souple (8) formant une jupe délimitant l'ouverture (7), ladite membrane (8) étant constituée dudit matériau élastique souple et étant moulée d'une pièce avec le restant du coussinet (5),
- le corps de masque (1) a une forme générale tridimensionnelle triangulaire et le masque est dimensionné pour s'adapter à la région nasale d'un bébé ou d'un enfant et
- un élément annulaire rigide (9) comprenant un polymère rigide est agencé dans l'orifice d'entrée (4) du corps de masque (1), ledit orifice d'entrée (4) étant agencé au centre du corps de masque (1) et la tête sphérique rigide (14) de l'élément de raccordement (12, 12a, 12b) étant fixé à rotation à l'élément annulaire rigide (9) agencé dans l'orifice d'entrée (4).

2. Masque selon la revendication 1, **caractérisé en ce que** le polymère rigide est une matière plastique.

3. Masque selon l'une quelconque des revendications précédentes, **caractérisé en ce que** l'élément de raccordement tubulaire (12, 12a, 12b) a une forme coudée et/ou comprend des trous de ventilation (17).

4. Masque selon l'une quelconque des revendications précédentes, **caractérisé en ce que** l'élément de raccordement tubulaire (12, 12a, 12b) comprend un orifice d'entrée secondaire (16) porté par un port d'entrée (15) et qui est en communication fluidique avec le passage à gaz interne traversant ledit élément de raccordement (12, 12a, 12b).

5. Masque selon l'une quelconque des revendications précédentes, **caractérisé en ce que** la forme générale tridimensionnelle triangulaire du corps de masque (1) comprend trois zones angulaires (1a-1c) formant les trois coins de la forme triangulaire du corps de masque (1), le bras de support (3) faisant saillie vers le haut de l'une (1a) desdites trois zones angulaires (1a-1c) dudit corps de masque (1), et les deux bras latéraux (2a, 2b) font saillie latéralement des deux autres zones angulaires (1b, 1c) dudit corps de masque (1).

6. Masque selon l'une quelconque des revendications précédentes, **caractérisé en ce que** le matériau élastique souple est une silicone.

7. Masque selon l'une quelconque des revendications précédentes, **caractérisé en ce que** l'ouverture (7) du coussinet (5) a une forme triangulaire.

8. Masque selon l'une quelconque des revendications précédentes, **caractérisé en ce que** la membrane (8) et le coussinet (5) comprennent une région de pont nasal supérieure, une région inférieure et deux régions latérales raccordant la région du pont nasal et la région inférieure de sorte que, lorsque le masque est porté par le patient, la région de pont nasal supérieure soit en contact avec le pont nasal du patient, que la région inférieure soit en contact avec la zone comprise entre le nez et la lèvre supérieure du patient et que les régions latérales opposées soient en contact avec les régions d'ailes du nez du patient.

9. Masque selon l'une quelconque des revendications précédentes, **caractérisé en ce qu'**il comprend en outre un casque comprenant des sangles.

10. Ensemble comprenant un dispositif de fourniture de gaz et un masque respiratoire pédiatrique selon l'une quelconque des revendications précédentes.

11. Ensemble selon la revendication 10, **caractérisé en ce que** le dispositif de fourniture de gaz est raccordé au masque respiratoire pédiatrique au moyen d'une conduite de gaz (13).
